(19) Europäisches Patentamt
European Patent Office
Office européen des brevets

(11) Veröffentlichungsnummer : **0 046 186**
**B1**

(12) # EUROPÄISCHE PATENTSCHRIFT

(45) Veröffentlichungstag der Patentschrift : **02.05.84**

(51) Int. Cl.³ : **C 12 P 13/04**, C 12 N 1/20// C12R1/01

(21) Anmeldenummer : **81105379.2**

(22) Anmeldetag : **10.07.81**

(54) **Verfahren zur Herstellung von D-N-Carbamoyl-alpha-aminosäuren und Mikroorganismen dafür.**

(30) Priorität : **18.08.80 DE 3031151**

(43) Veröffentlichungstag der Anmeldung : **24.02.82 Patentblatt 82/08**

(45) Bekanntmachung des Hinweises auf die Patenterteilung : **02.05.84 Patentblatt 84/18**

(84) Benannte Vertragsstaaten : **AT BE CH DE FR GB IT LI LU NL SE**

(56) Entgegenhaltungen :
**BE-A- 881 547**
**DE-A- 2 811 303**

(73) Patentinhaber : **BASF Aktiengesellschaft**
**Carl-Bosch-Strasse 38**
**D-6700 Ludwigshafen (DE)**

(72) Erfinder : **Lungershausen, Rolf, Dr.**
**Prager Strasse 29**
**D-6700 Ludwigshafen (DE)**
Erfinder : **Martin, Christoph, Dr.**
**Kolpingstrasse 6**
**D-6800 Mannheim (DE)**
Erfinder : **Marcinowski, Stefan, Dr.**
**Kopernikusstrasse 49**
**D-6700 Ludwigshafen (DE)**
Erfinder : **Siegel, Hardo, Dr.**
**Hans-Purrmann-Allee 25**
**D-6720 Speyer (DE)**
Erfinder : **Kuesters, Werner, Dr.**
**Osloer Weg 46**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 046 186 B1

Verfahren zur Herstellung von D-N-Carbamoyl-α-aminosäuren und Mikroorganismen dafür

Die Erfindung betrifft ein Verfahren zur Herstellung von D-N-Carbamoyl-α-aminosäuren aus Hydantoinen und Mikroorganismen dafür.

D-N-Carbamoyl-α-aminosäuren sind wichtige Zwischenprodukte zur Herstellung von D-Aminosäuren, welche wertvolle Ausgangsstoffe für Synthesen von Penicillinen und Cephalosporinen sind. Sie wurden bislang synthetisch oder biosynthetisch hergestellt. Bei der synthetischen Herstellung erhält man D,L-N-Carbamoyl-α-aminosäuren, die in die Antipoden getrennt werden müssen. Die Trennung in die Antipoden ist aufwendig und liefert nur Ausbeuten von maximal 50 %.

Optisch reine Carbamoylaminosäuren können durch enzymatische Spaltung von Hydantoinen mit Hilfe einer Dihydropyrimidinase erhalten werden (DE-OS 24 22 737). Die Reaktion läuft jedoch relativ langsam und liefert daher eine schlechte Raum-Zeit-Ausbeute. Zudem steht das Enzym nur in beschränker Menge zur Verfügung.

Gemäß der DE-OS 26 31 048 kann man razemische Gemische von Hydantoinen in optisch aktive Aminosäuren mit Hilfe von Mikroorganismen der Art Pseudomonas umwandeln. Diese Umsetzung erfolgt bei Temperaturen von etwa 30 °C und gelingt nur in ziemlich verdünnten Lösungen, so daß die Raum-Zeit-Ausbeute ebenfalls schlecht ist.

Gegenstand der Erfindung ist ein Verfahren zur Herstellung von D-Carbamoyl-α-aminosäuren der Formel I

$$H_2N-CO-NH-\overset{\overset{R}{|}}{C}H-COOH \qquad (I)$$

worin R eine Alkylgruppe mit 1 bis 4 Kohlenstoffatomen, bei der ein Wasserstoffatom durch eine $NH_2$-, OH-, oder SH-Gruppe substituiert sein kann, eine Benzylgruppe, bei der ein oder zwei Wasserstoffatome durch OH-Gruppen substituiert sein können, oder eine Phenylgruppe, bei der ein oder zwei Wasserstoffatome durch OH- oder $C_{1-4}$-Alkoxygruppe substituiert sein können, bedeutet, durch enzymatische Hydrolyse von Hydantoinen der Formel II

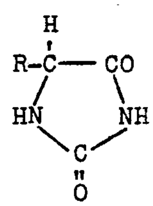

(II)

worin R die oben angegebene Bedeutung hat, dadurch gekennzeichnet, daß man die enzymatische Umwandlung mit Hilfe thermophiler, nicht sporenbildender, hydantoinspaltender Mikroorganismen oder daraus gewonnenen Extrakten durchführt.

Weiter betrifft die Erfindung thermophile, nicht sporenbildende, hydantoinspaltende Mikroorganismen oder daraus hergestellte Extrakte, die zur Herstellung von D-Carbamoyl-α-aminosäuren aus Hydantoinen geeignet sind.

Die thermophilen, nicht sporenbildenden, hydantoinspaltenden Mikroorganismen lassen sich wie folgt gewinnen :

Proben von Wasser, welches längere Zeit höherer Temperatur ausgesetzt ist — beispielsweise aus heißen Quellen aus Island oder dem Yellowstone Park (USA) — werden durch Membranfilter gesaugt. Letzere werden auf einem 30 min bei 121 °C steriliserten Nähragar folgender Zusammensetzung gelegt :

| | |
|---|---|
| Mineralsalzlösung nach Castenholz (Nature *215*), 1285 (1967) | 1 000 ml |
| Hefeextrakt | 2,5 g |
| Trypton | 2,5 g |
| Agar-Agar | 30,0 g |
| Natronlauge | bis pH 8,2 |

Die nach der Inkubation bei 60 °C auf dem Membranfilter wachsenden Kolonien werden isoliert und in Reinkultur gebracht. Zur Prüfung auf hydantoinhydrolase-Aktivität werden die so erhaltenen thermophilen, nicht sporenbildenden Bakterien in einer Lösung von 10 g/l $KH_2PO_4$, 75 ml/l 1 N NaOH und 10 g/l Methyl-thioethylhydantoin 24 Stunden bei 60 °C inkubiert. Die einzelnen Reaktionsgemische werden auf einen Gehalt an N-Carbamoylaminosäure untersucht. Ist das Ergebnis positiv, wird die Säure aus der Mischung isoliert und polarimetrisch auf optische Reinheit geprüft. Ist letztere gegeben, so liegt ein brauchbarer Mikroorganismus vor.

Zwei so erhaltene Stämme wurden beim Centraal Bureau voor Schimmelcultures (CBS) in Baarn/NL

unter den Nummern 303.80 am 11.4.1980 und 363.80 am 4.8.1980 hinterlegt. Sie haben folgende Eigenschaften :

| | CBS 303.80 | CBS 363.80 |
|---|---|---|
| Koloniemorphologie | rot pigmentiert, runde Kolonien | rotpigmentiert, runde Kolonien |
| Sporenbildung | negativ | negativ |
| Zellmorphologie | dünne Stäbchen, unbeweglich | unbewegliche Stäbchen |
| Sauerstoffverhältnis | obligat aerob | obligat aerob |
| Katalase | vorhanden | vorhanden |
| Oxidase | vorhanden | negativ |
| Gram-Färbung | gram-negativ | gram-negativ |
| Wachstumstemperatur | Min. 40°C, Opt. 60°C<br>Max. wenig oberhalb 60°C | 40 bis 60°C. |
| Wachstum mit: | Saccharose, Galaktose, Lactose | Saccharose, Glutamat, Acetat, Malat |
| kein Wachstum mit: | Glucose, Xylose, Glutamat, Acetat, Malat, Succinat, Glutarat | Galactose, Lactose, Glucose, Xylose, Succinat, Glutarat |
| Lackmusmilch | kein Abbau | kein Abbau, Lackmus-Reduktion |
| Stärke-Abbau | kein Abbau | kein Abbau |
| Casein-Abbau | negativ | positiv |
| Wachstum in 3 % NaCl-Brühe | negativ | negativ |

Beispiele für 5-substituierte Hydantoine, die mit den erfindungsgemäßen Bakterien zu N-Carbamoyl-D-Aminosäuren hydrolysiert werden können, sind in Tabelle 1 aufgeführt.

Tabelle 1

| Substrat | Produkt |
|---|---|
| 5-Methylhydantoin | D-N-Carbamoylalanin |
| 5-Ethylhydantoin | D-N-Carbamoyl-α-aminobuttersäure |
| 5-(2-Methyl-thioethyl)-hydantoin | D-N-Carbamoylmethionin |
| 5-Benzylhydantoin | D-N-Carbamoylphenylalanin |
| 5-Phenylhydantoin | D-N-Carbamoylphenylglycin |
| 5-(p-Hydroxiphenyl)-hydantoin | D-N-Carbamoyl-p-Hydroxiphenylglycin |
| 5-(p-Methoxiphenyl)-hydantoin | D-N-Carbamoyl-p-methoxiphenylglycin |
| 5-(p-t-Butoxiphenyl)-hydantoin | D-N-Carbamoyl-p-t-butoxiphenylglycin |
| 5-(p-Acetoxiphenyl)-hydantoin | D-N-Carbamoyl-p-acetoxiphenylglycin |

Das erfindungsgemäße Verfahren wird vorzugsweise bei 40 bis 90 °C und einem pH-Wert von 7 bis 10 durchgeführt. Für das Verfahren können die erfindungsgemäßen Bakterien bzw. daraus abgeleitete Mutanten in freier oder immobilisierter Form verwendet werden. Es können auch Extrakte aus den Mikroorganismen eingesetzt werden. Die Extrakte lassen sich beispielsweise wie folgt herstellen :

Eine Zellsuspension in Puffer wird unter Eiskühlung 5 bis 10 min mit Ultraschall behandelt. Nach Abzentrifugieren der Zelltrümmer ist das hydantoinspaltende Enzym im Überstand gelöst. Dieser Überstand kann direkt als Rohextrakt für das erfindungsgemäße Verfahren eingesetzt werden.

Das erfindungsgemäße Verfahren bzw. die neuen Mikroorganismen besitzen folgende Vorteile :

1. Die Kultivierung nicht sporenbildender thermophiler Mikroorganismen ist einfacher als die sporenbildender thermophiler Mirkoorganismen (z. B. Bacillus-Arten, vgl. DE-OS 28 11 303), da bei letzteren die Fermentation vor Eintritt der Sporulation abgebrochen werden muß. Bei den erfindungsgemäßen Mikroorganismen besteht die Gefahr einer Sporulation nicht, hier liegen immer enzymatisch aktive Zellen vor.

2. Die Zellausbeute ist bei nicht sporenbildenden Organismen höher als bei entsprechenden Sporenbildnern.

3. Die thermophilen, nicht sporenbildenden Bakterien erlauben es, die Umsetzung bei höheren Temperaturen als in der Literatur beschrieben durchzuführen. Dies hat mehrere Vorteile :

a) höhere Löslichkeit der Hydantoine,
b) schnellere Nachracemisierung der Hydantoine,
c) schnellere enzymatische Umsetzung der Hydantoine zu den D-Carbamoylaminosäuren.

4. Die für die Hydantoinhydrolyse eingesetzten thermophilen nicht sporenbildenten Stämme tolerieren wesentlich höhere Substratkonzentrationen als die bisher beschriebenen Organismen. Somit sind die Raum-Zeit-Ausbeuten höher.

Beispiel 1

Drei 1-1-Erlenmeyer-Kolben mit je 170 ml sterilem Medium (Zusammensetzung : Mineralsalzlösung nach Castenholz (Nature *215*, 1 285 (1967)), 0,258 g/l $Na_3PO_4 \cdot 12H_2O$, 10 g/l Hefeextrakt, 2,5 g/l Trypton, pH 7,0) werden mit Schrägagarkulturen des Stamms CBS 303.80 beimpft und 46 Stunden bei 60 °C unter Schütteln (200 Upm) inkubiert.

Danach wird ein Fermenter, der 20 l steriles Medium obiger Zusammensetzung und 5 ml Antischaummittel enthält, mit diesen 3 Vorkulturen beimpft. Es wird unter folgenden Bedingungen fermentiert :

Temperatur : 60 °C
pH : 7,0, eingehalten durch Zugabe von 2 M $H_3PO_4$
Belüftung : 0,1 VVM Luft
Drehzahl : 500 Upm

Nach 25,5 Stunden Fermentationsdauer wird 1 l der Kulturbrühe entnommen (11,4 g Bakterienfeuchtmasse enthaltend), mit 1 g Polyoxiethylen-20-cetylether sowie 17,6 g 5-Phenylhydantoin versetzt und folgendermaßen weiterinkubiert :

Temperatur : 60 °C
pH : 8,5, eingehalten durch Zugabe von 5 N NaOH
Belüftung : 0,1 VVM Stickstoff
Drehzahl : 200 Upm

Nach 7 Stunden Inkubationszeit wird der Ansatz zentrifugiert und das D-N-Carbamoylphenylglycin durch Ansäuern des Überstands mit 10 %iger HCl auf pH 2,5 ausgefällt.

Nach Umkristallisation in Aceton beträgt der spez. Drehwert $[\alpha]_D^{20°C} = -138{,}8°$ (C = 1 in 1 M $NH_3$), der Schmelzpunkt 193 °C.

Beispiel 2

Der Stamm CBS 303.80 wird, wie in Beispiel 1 beschrieben, gezüchtet, jedoch werden während der Anzucht der Zellmasse die Drehzahl und Belüftung so geregelt, daß die $O_2$-Konzentration im Nährmedium ständig 10 % des durch Belüftung mit Luft maximal erreichbaren Wertes beträgt. Nach einer Fermentationsdauer von 23 Stunden wird die Zellmasse durch Zentrifugation gewonnen, der Überstand verworfen.

50 g feuchte Zellmasse werden in 1 l Medium folgender Zusammensetzung suspendiert :

2 g/l $KH_2PO_4$
100 g/l 5-Phenylhydantoin
1 g/l entionisiertes Wasser

Der pH-Wert wird mit 5 N NaOH auf 8,5 eingestellt und konstant gehalten. Der Ansatz wird auf 60 °C temperiert, mit 300 Upm gerührt und mit 0,1 VVM $N_2$ begast.

Nach 5 Stunden beträgt die Ausbeute an N-Carbamoylphenylglycin 94,5 %. Der Ansatz wird wie in Beispiel 1 aufgearbeitet. Das Umsetzungsprodukt ist wieder optisch reines D-N-Carbamoylphenylglycin.

Beispiel 3

Zellmasse des Stamms CBS 303.80 wird wie in Beispiel 2 gewonnen und anschließend bei 40 °C im Vakuum bis zur Gewichtskonstanz getrocknet. 5,75 g dieses Zelltrockenpulvers werden in 500 ml Medium mit 2 g/l $KH_2PO_4$ und 100 g/l 5-Phenylhydantoin wie in Beispiel 2 inkubiert. Die Ausbeute an optisch reinem D-N-Carbamoylphenylglycin beträgt nach 6 Stunden 86 %.

Beispiel 4

25 g feuchte Zellmasse des Stamms CBS 303.80, die wie in Beispiel 2 beschrieben erhalten wurde, wird in 500 ml des in Beispiel 2 beschriebenen Mediums suspendiert, 9 min bei 4 °C beschallt (20 kHz) und anschließend bei 60 °C, 300 Upm, 0,1 VVM $N_2$ und pH 8,5 inkubiert. Die Ausbeute an optisch reinem D-N-Carbamoylphenylglycin beträgt nach 6 Stunden 69 %.

Beispiel 5

25 g feuchte Zellmasse des Stamms CBS 303.08, die wie in Beispiel 2 beschrieben erhalten wurde, wird in 500 ml Medium folgender Zusammensetzung bei 60 °C, 300 Upm, 0,1 VVM $N_2$ und pH 8,5 inkubiert :

50 g 5-Penylhydantoin
1 g $KH_2PO_4$
0,5 g Polyoxiethylen-20-cetylether

entionisiertes Wasser ad 500 ml.

Die Ausbeute an optisch reinem D-N-Carbamoylphenylglycin beträgt nach 7 Stunden 86 %.

Beispiel 6

60 g Zellfeuchtmasse, die wie in Beispiel 2 hergestellt wurde, wird in 1 l Medium folgender Zusammensetzung suspendiert :

10 g/l $KH_2PO_4$
1 g/l Polyoxiethylen-20-cetylether
50 g/l 5-(p-Methoxiphenyl)-hydantoin

Die Umsetzung erfolgt unter folgenden Bedingungen :

Temperatur 60 °C
pH 8,5 konstant gehalten durch Zugabe von 5 M NaOH
0,1 VVM $N_2$.

Nach 22 Stunden wird der Versuch abgebrochen und das entstandene D-N-Carbamoyl-p-methoxiphenylglycin wie in Beispiel 1 isoliert, Ausbeute : 92 %, Schmelzpunkt : 212 bis 213 °C, $[\alpha]_D^{20\,°C} = -138,7$ (C = 1 in 1 M $NH_3$)

Die Verbindung ist in ihren Eigenschaften identisch mit D-N-Carbamoyl-p-methoxiphenylglycin.

Beispiel 7

20 g feuchte Zellmasse des Stammes CBS 303.80, die wie in Beispiel 2 beschrieben erhalten wurde, wird in 500 ml Medium folgender Zusammensetzung bei 60 °C, 300 Upm, 0,1 VVM $N_2$ und pH 8,5 inkubiert :

20 g p-tert.-Butoxiphenylhydantoin
1 g $KH_2PO_4$
0,5 g Polyoxiethylen-20-cetylether

entionisiertes Wasser ad 500 ml.

Die Ausbeute an optisch reinem N-Carbamoyl-D-p-tert.-Butoxiphenylglycin beträgt nach 26 Stunden 39 %.

Beispiel 8

20 g feuchte Zellmasse des Stammes CBS 303.80, die wie in Beispiel 2 beschrieben hergestellt wurde, wird in 500 ml Medium folgender Zusammensetzung bei 60 °C, 300 Upm, 0,1 VVM $N_2$ und pH 8,5 inkubiert :

10 g 5-Benzylhydantoin
1 g $KH_2PO_4$
0,5 g Polyoxiethylen-20-cetylether

entionisiertes Wasser ad 500 ml.

Die Ausbeute an optisch reinem N-Carbamoyl-D-phenylalanin beträgt nach 25,5 Stunden 30 %.

Beispiel 9

0,9 g feuchte Zellmasse des Stammes CBS 363.80, die wie in Beispiel 2 beschrieben hergestellt wurde, wird in 100 ml Medium folgender Zusammensetzung bei 60 °C und pH 8,77 inkubiert :

0,5 g 5-Methylthioethylhydantoin
7 g $K_2HPO_4$

entionisiertes Wasser ad 100 ml.

Nach 48 Stunden beträgt die Ausbeute an optisch reinem N-Carbamoyl-D-methionin über 90 %.

Beispiel 10

Unter Bedingungen, wie sie in Beispiel 8 beschrieben wurden, werden 20 g 5-Methylthioethylhydantoin mit 25 g feuchter Zellmasse umgesetzt. Die Ausbeute an optisch reinem N-Carbamoyl-D-methionin beträgt nach 6 Stunden 87,7 %.

Beispiel 11

Eine 20 %ige Suspension von Zellen (Feuchtgewicht), die wie in Beispiel 2 beschrieben gezüchtet wurden, in 0,2 M Tris/HCl pH 8,0 wurde 3 × 3 min beschallt. Nach Zentrifugation (10 000 g) wurden 72 ml des Überstands mit 18 ml einer 1 %igen wäßrigen Lösung von Cetylpyridiniumchlorid versetzt. Das Zentrifugat dieses Fällungsschrittes stellt die für die Umsetzung verwendete Hydantoinase-Rohpräparation dar. Die Umsetzung wurde folgendermaßen durchgeführt :

90 ml Hydantoinase-Rohpräparation
410 ml entionisiertes Wasser
50 g Phenylhydantoin
pH 8,5 (eingestellt mit NaOH)
Temp. 60 °C
300 Upm
Begasung mit $N_2$

Die Ausbeute an optisch reinem D-N-Carbamoylphenylglycin betrug nach 24 Stunden 94,1 %.

Beispiel 12

100 ml einer wie in Beispiel 11 hergestellten Hydantoinase-Rohpräparation wurden mit 100 ml entionisiertem Wasser verdünnt und bei 4 °C mit 30 g feuchter DE-52-Cellulose (Whatman ; äquilibriert mit 100 mM Tris/HCl, pH 8,0) geschüttelt. Nach 2 Stunden war die gesamte Hydantoinase-Aktivität am Ionenaustauscher adsorbiert ; er wurde abgenutscht, mit 50 ml 50 mM Tris/HCl pH 8,0 gewaschen ; im Waschwasser konnte keine Hydantoinase-Aktivität nachgewiesen werden. Diese trägerfixierte Rohpräparation wurde mit 50 g Phenylhydantoin in 450 ml entionisiertem Wasser bei pH 8,5 und 60 °C unter Begasung mit $N_2$ inkubiert. Die Ausbeute an optisch reinem D-N-Carbamoylphenylglycin betrug nach 6,5 Stunden 94,9 %.

Beispiel 13

Zu einer Suspension von 7,5 g Acrylamid, 0,4 g N,N'-Methylenbisacrylamid und 10 g feuchter Zellmasse aus Beispiel 2 in 50 ml phys. Kochsalzlösung wurde unter Begasung mit $N_2$ 60 µl Tetramethylethylendiamin und 2 ml einer 10 %igen wäßrigen $(NH_4)_2S_2O_8$-Lösung zugegeben und bei Raumtemperatur 30 min stehen gelassen. Das so gebildete Gel wurde in einem Mixer zerkleinert, anschließend 5 mal mit je 100 ml physiologischer Kochsalzlösung gewaschen. Die so behandelten Gelpartikel wurden in 600 ml Wasser suspendiert, die 1 g $KH_2PO_4$ und 10 g 5-Phenyhydantoin enthielten. Die Ausbeute an N-Carbamoyl-D-phenylglycin betrug nach 6,5 Stunden 97,3 %. Zu dieser Lösung wurden weitere 30 g Phenylhydantoin gegeben. Nach weiteren 15,5 Stunden betrug die Ausbeute 94,5 %.

Beispiel 14

Zellmasse des Stammes CBS 303.80, die wie in Beispiel 2 beschrieben angezogen wurde, wurde bei 40, 50, 60, 70, 80 bzw. 90 °C 30 min in folgendem Puffer inkubiert : 10 g/l $KH_2PO_4$, 1 g/l Polyoxiethylen-20-cetylether, 10 g/l Methylthioethylhydantoin, 10 g/l Bakterientrockenmasse, pH eingestellt mit NaOH auf 8,5. Anschließend wurde der Gehalt an N-Carbamoyl-D-methionin bestimmt :

| Temperatur (°C) | Ausbeute an N-Carbamoyl-D-methionin (%) |
|---|---|
| 40 | 43,4 |
| 50 | 48,0 |
| 60 | 55,9 |
| 70 | 61,4 |
| 80 | 70,6 |
| 90 | 34,1 |

Beispiel 15

Zellmasse aus Beispiel 2 wurde 30 min bei 60 °C und pH-Werten zwischen pH 7,0 und 10 in folgendem Puffer inkubiert (dabei wurde der pH-Wert durch Dosierung von NaOH auf dem gewünschten Wert gehalten) :

10 g/l Methylthioethylhydantoin, 10 g/l $KH_2PO_4$, 1 g/l Polyoxiethylen-20-cetylether, 10 g/l Bakterientrockenmasse. Die Ausbeute an N-Carbamoyl-D-methionin hing folgendermaßen vom pH-Wert ab :

| pH-Wert | Ausbeute an N-Carbamoyl-D-methionin (%) |
|---|---|
| 7,0 | 46,5 |
| 7,5 | 48,5 |
| 8,0 | 55,2 |
| 8,5 | 58,0 |
| 9,0 | 51,7 |
| 9,5 | 50,7 |
| 10,0 | 22,3 |

Beispiel 16

2,5 ml einer wie in Beispiel 11 hergestellten Hydantoinase-Rohpräparation werden mit 60 ml Wasser verdünnt. Die Mischung wurde mit 300 mg p-Hydroxiphenylhydantoin versetzt und unter $N_2$-Begasung bei 60 °C gerührt. Der pH-Wert wurde dabei durch Zugabe von 1 N Natronlauge auf 8,0 gehalten. Die Ausbeute an optisch reinem N-Carbamoyl-p-hydroxi-D-phenylglycin betrug nach 18 Stunden 92 %.

**Ansprüche**

1. Verfahren zur Herstellung von D-Carbamoyl-α-aminosäuren der Formel I

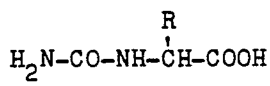

(I)

worin R eine Alkylgruppe mit 1 bis 4 Kohlenstoffatomen, bei der ein Wasserstoffatom durch eine $NH_2$-, OH- oder SH-Gruppe substituiert sein kann, eine Benzylgruppe, bei der ein oder zwei Wasserstoffatome durch OH-Gruppen substituiert sein können, oder eine Phenylgruppe, bei der ein oder zwei Wasserstoffatome durch OH- oder $C_{1-4}$-Alkoxygruppen substituiert sein können, bedeutet, durch enzymatische Hydrolyse von Hydantoinen der Formel II

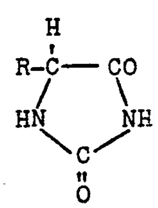

(II)

worin R die oben angegebene Bedeutung hat, dadurch gekennzeichnet, daß man die enzymatische Umwandlung mit Hilfe thermophiler, nicht sporenbildender, hydantoinspaltender Mikroorganismen oder daraus gewonnenen Extrakten durchführt.

2. Thermophile, nicht sporenbildende, hydantoinspaltende Mikroorganismen und daraus hergestellte hydantoinspaltende Extrakte.

**Claims**

1. A process for the preparation of a D-carbamyl-α-aminoacid of the formula I

$$H_2N{-}CO{-}NH{-}\overset{R}{\overset{|}{C}}H{-}COOH \qquad (I)$$

where R is alkyl of 1 to 4 carbon atoms, in which one hydrogen can be replaced by $NH_2$, OH or SH, or R is benzyl, in which one or two hydrogens can be replaced by OH, or R is phenyl, in which one or two hydrogens can be replaced by OH or $C_{1-4}$-alkoxy, by enzymatic hydrolysis of a hydantoin of the formula II

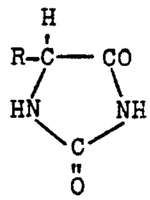

(II)

where R has the above meanings, wherein the enzymatic conversion is effected with the aid of thermophilic, non-sporulating, hydantoin-cleaving micro-organisms or of extracts obtained therefrom.

2. A thermophilic, non-sporulating, hydantoin-cleaving micro-organism, and the hydantoin-cleaving extracts prepared therefrom.

**Revendications**

1. Procédé de préparation de d-carbamoyl-α-aminoacides de la formule I

$$H_2N{-}CO{-}NH{-}\overset{R}{\overset{|}{C}}H{-}COOH \qquad (I)$$

dans laquelle R désigne un radical alkyle en $C_1$ à $C_4$, dont un atome d'hydrogène peut être substitué par un groupe $-NH_2$, —OH ou —SH, un groupe benzyle, dont un ou deux atomes d'hydrogène peuvent être substitués par des groupes —OH, ou un groupe phényle, dont un ou deux atomes d'hydrogène peuvent être substitués par des groupes —OH ou alcoxy en $C_1$ à $C_4$, par hydrolyse enzymatique d'hydantoïnes de la formule II

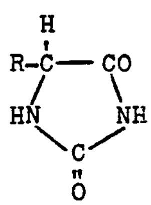

(II)

dans laquelle R possède la signification définie, caractérisé en ce que la réaction enzymatique est réalisée à l'aide de micro-organismes non sporulants thermophiles, capables de cliver les hydantoïnes, ou d'extraits de ceux-ci.

2. Micro-organismes non sporulants thermophiles, capables de cliver les hydantoïnes, et extraits préparés à partir de ceux-ci.